# EUROPEAN PATENT APPLICATION

(11) **EP 4 309 694 A1**
(43) Date of publication of application: **24.01.2024**
(21) Application number: 23186693.0
(22) Date of filing: 20.07.2023
(51) Int. Cl.: A61M 1/36, G16H 20/40, G16H 40/63, A61M 1/30

(54) **BLOOD PROCESSING AND TREATMENT SYSTEMS AND METHODS WITH PROCEDURE ESTIMATOR**

(30) Priority: 22.07.2022 US 202263391604 P
(71) Applicant: Fenwal, Inc., Lake Zurich, IL 60047 (US)
(72) Inventor: COULTAS, Gregory, Lake Zurich, 60047 (US); NGUYEN, Lan T., Lake Zurich, 60047 (US); CASE, Brian C., Lake Zurich, 60047 (US)
(74) Representative: Maikowski & Ninnemann Patentanwälte Partnerschaft mbB

(57) **Abstract**

Systems and methods for the optimization of blood collection and therapies using an automated blood cell separator are disclosed. The systems and methods calculate or determine recommended settings based on, among other things, one or more of historical collection data, set defaults, contamination limits, blood, and blood component characteristics.

## Description

### Field of the Disclosure

The present disclosure is directed to the processing, collection and/or treatment of blood and/or blood components. More particularly, the present disclosure is directed to blood processing and/or treatment systems and methods that include a procedure estimator for optimizing the collection of blood components and/or the treatment of a collected blood component. Even more particularly, the present disclosure is directed to systems and methods wherein a controller is configured to establish an optimized collection and/or therapeutic procedure based on one or more of blood component pre-counts, desired yields, historical data, component characteristics, intermediate results and the like.

### Background

Whole blood is made up of various cellular components such as red blood cells, white blood cells (or leukocytes) and platelets suspended in its liquid component, plasma. Whole blood can be separated into its constituent components (cellular or liquid), and the desired component can be collected so that it can be administered to a patient in need of that particular component. For example, mononuclear cells (MNCs), primarily lymphocytes and monocytes, can be removed from the whole blood of a patient, collected, and treated for use in therapy. One such therapy is a procedure commonly referred to as extracorporeal photopheresis, or ECP. In ECP, MNCs are treated with a photosensitizing agent and subsequently irradiated with specified wavelengths of light to achieve a desired effect and returned to the patient for the treatment of various blood diseases to, e.g., eliminate immunogenicity in cells, inactivate or kill selected cells, or activate desirable immune responses.

More specifically, separated MNCs may be separated from whole blood, chemically treated with a light-activated agent, such as 8-methoxypsoralen (8-MOP, either added to the separated MNCs or administered in advance to the patient), exposed to ultraviolet light, and returned to the patient. The activated 8-methoxypsoralen crosslinks with the DNA in the exposed MNCs, ultimately resulting in apoptosis of the MNCs. The photochemically-damaged MNCs returned to the patient to induce cytotoxic effects on T-cell formation, resulting in an antitumor action. Examples of MNC collection and treatment for use in ECP are described in U.S. Patent No. 9,399,093, U.S. Patent No. 10,213,544, U.S. Patent No. 10,751,433, U.S. Patent No. 11,318,239.

Mononuclear cells (MNCs) may be collected for other reasons as well. For example, MNCs may serve as starting materials in the development and manufacture of drugs and other therapeutic agents. The collection of MNCs is described in U.S. Patent No. 6,027,657.

Other types of cells that may be useful in therapeutic treatments are stem cells. Stem cells are undifferentiated cells that can turn into specific cells. They have shown promise in several treatments including, without limitation, tissue regeneration and potentially in the treatment of disease. U.S. Patent No. 11,311,823, describes systems and methods for the collection of MNCs and peripheral blood stem cells.

Mononuclear cells may be collected by introducing whole blood into a centrifuge chamber wherein the whole blood is separated into its constituent components based on the size and densities of the different components, with the targeted component(s) being collected, and the non-target components either being returned to the patient or otherwise disposed of. Typical blood processing systems thus include a permanent, separator, such as a reusable centrifuge assembly containing the hardware (drive system, pumps, valve actuators, programmable controller, and the like) that spins and pumps the blood, and a disposable, sealed and sterile fluid processing assembly that is mounted cooperatively on the hardware. The centrifuge assembly spins a disposable centrifuge chamber in the fluid processing assembly during a collection procedure, thereby separating the blood into its constituent components such as MNCs and stem cells.

In view of the growing interest in using collected MNCs and stem cells in therapeutic and other treatments, and in the development of drugs and other beneficial agents, it would be desirable to optimize the procedures used to collect such cells. For example, with regard to stem cells, MNC and ECP procedures, it would be beneficial for the medical practitioner prescribing the collection or therapy to be able to target a selected amount of stem cells or mononuclear cells to be collected or treated. For MNC procedures, the medical practitioner may collect the cells for further processing wherein the downstream process may require a predetermined amount of cells with varying, albeit relatively minimal, levels of cellular contamination from red cells, platelets, granulocytes. For ECP procedures, the medical practitioner may seek to treat and reinfuse a desired amount of the patient's cells to optimize the treatment regimen.

The systems and methods described herein address many of these desired goals.

### Summary

There are several aspects of the present subject matter which may be embodied separately or together in the systems and methods described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a system for processing and/or treating blood and blood components is provided. The system includes a separator for separating blood into its components and a disposable fluid circuit associated with the separator. The system includes a controller configured to (i) direct the collection and/or treatment of blood components (ii) store information based on one or more of historical collection data, pre-determined default values or limits for certain components, selected blood component conditions and (iii) establish and/or calculate recommended procedure settings based on the stored information and other input information. The system further includes a user interface associated with the separator and coupled to the controller for entering information and displaying the established and/or calculated procedure settings.

In another aspect, a method for optimizing a blood component collection and/or treatment procedure in an automated blood cell processing system is provided. The method includes the steps of a) entering or retrieving selected product data into the processing system (b) calculating a collection efficiency based on historical data and/or default collection efficiencies (c) entering selected blood component conditions for a blood component collection and/or treatment procedure (d) entering or retrieving one or more of product contamination limits, blood conditions and (e) determining recommended procedure settings based on entered, retrieved or calculated values from (a)-(d) above.

### Brief Description of the Drawings

Figure 1 is a partial perspective view of a blood separator useful in the methods and systems described herein, according to an exemplary embodiment;
Figure 2 is a perspective view of a separation chamber of the processing set used with the separator of FIG. 1, according to an exemplary embodiment;
Figure 3 is a diagram of the fluid circuit useful in the collection, treatment, and reinfusion of target cells, according to an exemplary embodiment;
Figure 4 is a diagram generally showing the mechanical components of an extracorporeal photopheresis (ECP) treatment system, according to an exemplary embodiment;
Figure 5 is a schematic representation of a controller of a processing system including a blood separator in accordance with the present disclosure; and
Figure 6 is a flow chart setting forth the steps in a method for optimizing a collection of blood components and or the treatment of a collected blood component.

### Detailed Description of the Embodiments

Features, aspects, and advantages of the present embodiments will become apparent from the following description, appended claims, and the accompanying exemplary embodiments shown in the drawings, which are briefly described below.

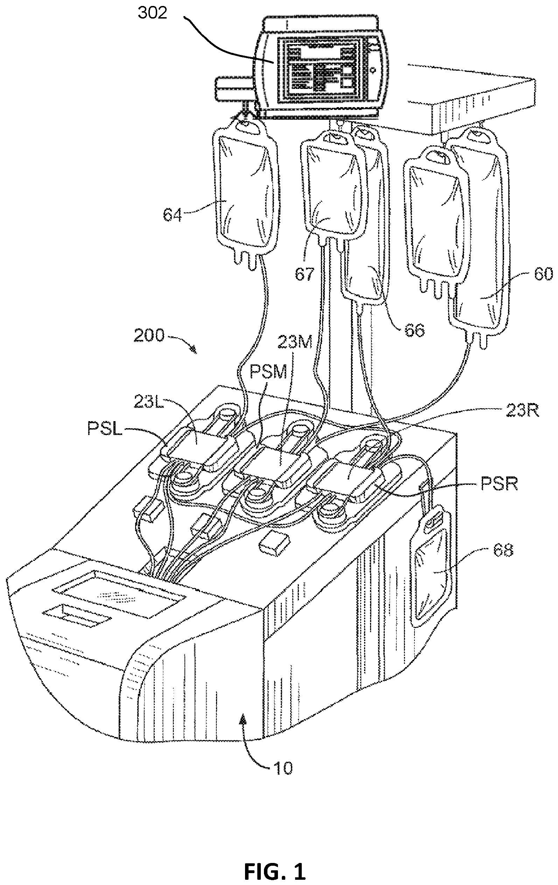

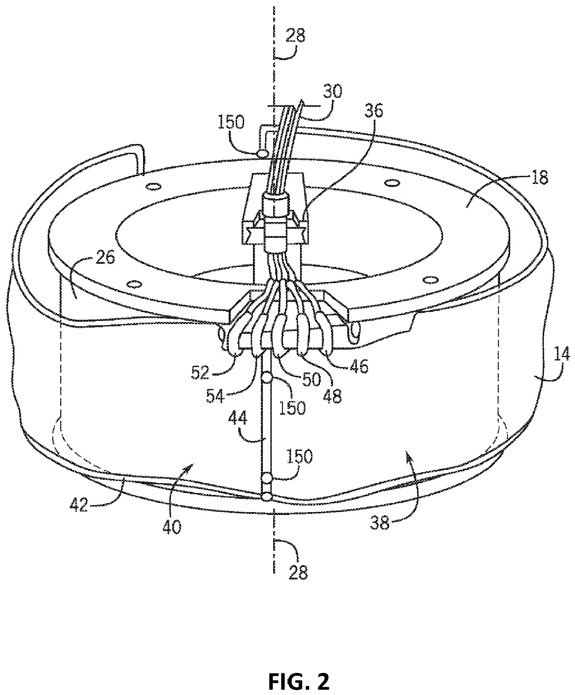

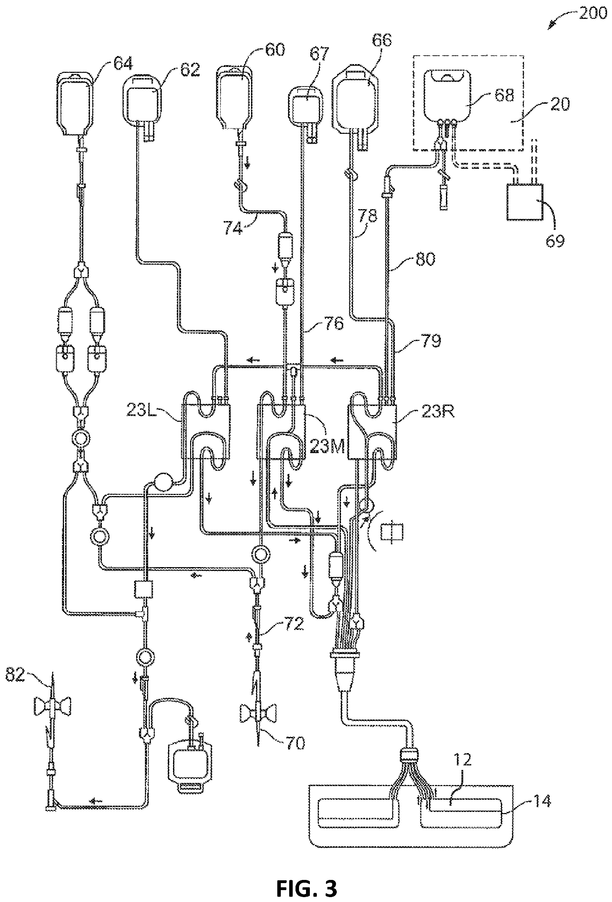

FIGS. 1-3 depict an automated separator 10 useful in the separation of blood into two or more components with fluid circuit 200 mounted thereon, the fluid circuit (FIG. 3) having a blood processing container 14 (FIG. 2) defining a separation chamber 12 suitable for harvesting mononuclear cells (MNC) from whole blood. As shown in FIG. 1, a disposable processing set or fluid circuit 200 (which includes container 14) may be mounted on the front panel of separator 10. The fluid circuit 200 may include a plurality of processing cassettes 23L, 23M and 23R with tubing loops for association with peristaltic pump stations PSL, PSM and PSR on separator 10. Each pump station (PS) may include two peristaltic pumps, for a total of six pumps in the circuit 200 (see FIG. 3). As described in U.S. Patent No. 6,027,657, valve stations are molded into the back side of the cassette body of cassettes 23L, 23M and 23R and are actuated by valve actuators. A flexible diaphragm covers and seals the back side of the cassette body. The left, middle, and right cassettes 23L, 23M, and 23R mate with pump stations (PSL, PSM, and PSR) on the separator 10.

Fluid circuit 200 may also include a network of tubing defining flow paths and pre-connected containers for establishing flow communication with a suitable blood source (for example the donor or patient or a container with previously collected blood) and for processing and collecting fluids and blood and blood components, as shown in FIG. 3. Thus, it is to be understood that the method as described in the following can be applied ex vivo (i.e., without a patient being in direct flow communication with the fluid circuit 200). As seen in FIGS. 1 and 3, disposable processing set 200 may include a container 60 for supplying anticoagulant, a waste container 62 for collecting waste from one or more steps in the process for treating and washing mononuclear cells, a container 64 for holding saline or other wash or resuspension medium, a container 66 for collecting plasma, a container 68 for collecting the desired blood component e.g., mononuclear cells and, optionally, container 69 for holding the photoactivation agent (in one exemplary embodiment directed to extracorporeal photopheresis (ECP).

With reference to FIG. 3, fluid circuit 200 may include inlet line 72, an anticoagulant (AC) line 74 for delivering AC from container 60, an RBC line 76 for conveying red blood cells from chamber 12 of container 14 to container 67, a platelet poor plasma (PPP) line 78 for conveying PPP to container 66 and line 80 for conveying mononuclear cells to and from blood processing container 14 and collection/illumination container 68. The blood processing set may include one or more venipuncture needle(s) for accessing the blood source (for example the circulatory system of the patient or a container with previously collected blood). As shown in FIG. 3, fluid circuit 200 may include inlet needle 70 and return needle 82. In an alternative embodiment, a single needle may serve as both the inlet and outlet needle.

Fluid flow through fluid circuit 200 may be driven, controlled and adjusted by a microprocessor-based controller (described in greater detail below) in cooperation with the valves, pumps, weight scales and sensors of device 10 and fluid circuit 200, the details of which are described in the aforementioned U.S. Pat. No. 6,027,657, , although any suitable controller may be used. Fluid flow through the circuit 200 may also be discerned and visualized by an optical sensor and/or hematocrit sensor located at or near separation chamber 12 and part of the device 10 to sense and quantify MNCs, RBCs, and/or plasma for harvesting. Details regarding a suitable optical sensor and method of blood component visualization are also described in U.S. Pat. No. 6,027,657. In accordance with the present disclosure, the optical sensor, certain valves within cassettes 23L, 23M and 23R and pumps work (under the direction of the controller) to process pre-determined Offset Volumes for certain components, described in greater detail below.

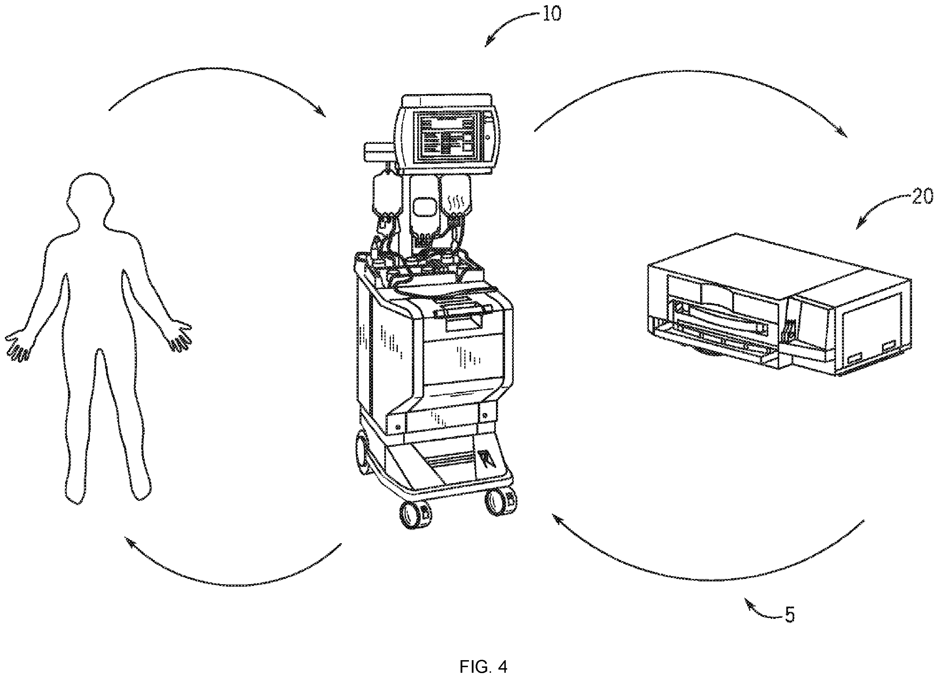

In accordance with the present disclosure, the fluid circuit may be further adapted for association with the irradiation device 20, as shown in Fig.4 for carrying out a therapeutic ECP procedure. One example of a suitable irradiation device is described in U.S. Pat. No. 7,433,030, although any suitable irradiation device may be used. The irradiation device 20 may include a tray or other holder for receiving one or more containers during treatment.

In accordance with the ECP procedure, whole blood may first be withdrawn from the blood source through inlet needle 70 and introduced into the separation chamber 12 of container 14 of processing set 200, where the whole blood is subjected to a centrifugal field. The centrifugal field may separate the target cell population, i.e., mononuclear cells, from a red blood cell constituent and a platelet/plasma. The components such as red blood cells and platelets may be returned to the blood source or may be diverted to a container (e.g., container 67) for further processing. Collection of the mononuclear cells may proceed in one or more cycles, with the number of processing cycles conducted in a given therapeutic procedure depending upon the total yield of MNCs to be collected. Container 68 may also serve as the illumination container, and the illumination container 68 may be pre-attached to and with integral the disposable set 200. Container 68 may be housed within an adjacent separately housed irradiation device 20, thereby eliminating the step of having the operator place container 68 into irradiation device 20. The tubing leading to and/or from container 68 in fluid circuit 200 may be of a sufficient length to reach an irradiation device 20 that is adjacent to but housed separately from the separation device.

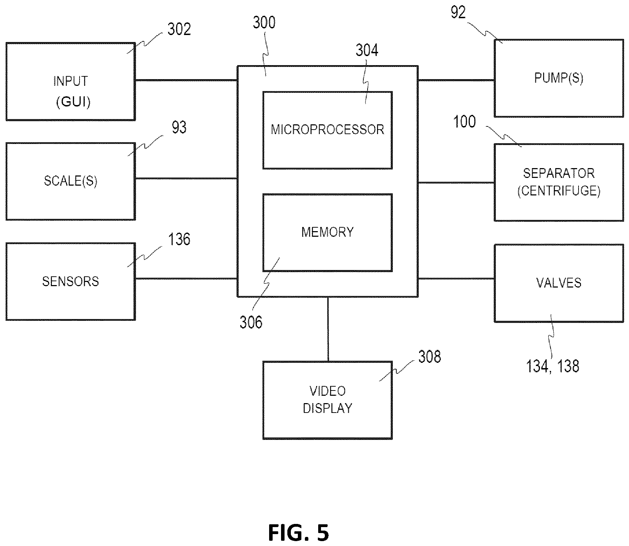

According to an aspect of the present disclosure, the blood separation system includes a controller 300, schematically shown in Fig. 5, which is suitably configured and/or programmed to control operation of the separation system and, as described below, receive inputs of data and output recommended procedure settings. The controller 300 may include a microprocessor 304 (which, in fact may include multiple physical and/or virtual processors). According to other embodiments, the controller 300 may include one or more electrical circuits designed to carry out the actions described herein. In fact, the controller 300 may include a microprocessor and other circuits or circuitry. In addition, the controller 300 may include one or more memories 306. The instructions by which the microprocessor 304 is programmed may be stored on the memory 306 associated with the microprocessor 304, which memory/memories 306 may include one or more tangible non-transitory computer readable memories, having computer executable instructions stored thereon, which when executed by the microprocessor 304, may cause the microprocessors 304 to carry out one or more actions as described below.

As is also illustrated in Fig. 5, the controller 300 may be coupled to one or more of the structures described above, for example to receive information (e.g., in the form of signals) from these structures or to provide commands (e.g., in the form of signals) to these structures to control the operation of the structures. As illustrated, the controller 300 may be coupled to scales, the sensors 136 and the at least one input 302 to receive information from those devices. Additionally, the controller 300 may be coupled to pumps 92 of pump stations PSL, PSM, PSR, clamps, valves 134, 138 (associated with cassettes 23L, 23M, 23R) and the drive or centrifuge separator to provide commands to those devices to control their operation. The controller 300 may be directly electrically connected to these structures to be coupled to them, or the controller 300 may be directly connected to other intermediate equipment that is directly connected to these structures to be coupled to them.

The at least one input or user interface 302 (also see Fig. 1) may include a number of different devices according to the embodiments described herein. For example, the input 302 could include a keyboard or keypad by which a user may provide information and/or instructions to the controller 300. Alternatively, the input 302 may be a touch screen, such as may be used in conjunction with or combined with a video display 308. The input could also include a reader or scanner, such as a barcode reader or scanner or an RFID reader. The assembly of the input/touch screen 302 and video display 308 may be one of the afore-mentioned structures to which the controller 300 is coupled from which the controller 300 receives and displays information and to which the controller 300 provides commands. According to still other embodiments, the input 302 may be in the form of computer equipment that permits the blood separation system including the controller 300 to communicate (whether via wires, cables, etc. or wirelessly) with other systems over a local network, or with other cell processing systems or other computer equipment (e.g., a server) over local networks, wide area networks, or the Internet. According to such an embodiment, the input may include an internal transmitter/receiver device.

The controller is configured and/or programmed to execute at least one fluid processing application but, more advantageously, is configured and/or programmed to execute a variety of different fluid processing applications.

More particularly, in carrying out any one of these blood processing applications, the controller is configured and/or programmed to control the flow of a fluid through a separator and adjusts the speed of the flow into and out of the separator. This may include the separator to operate at a particular rotational speed and instructing a first pump to convey fluid through the inlet of the separator at a particular flow rate and instructing a second pump to convey fluid out of the outlet of the separator at a particular flow rate. Any known method of adjusting speed of the flow into and out of the separator may be used. Hence, while it may be described herein that a particular component of the blood separation system performs a particular function, it should be understood that the component is being controlled by the controller to perform that function. For example, the controller may instruct one of the pumps to cause blood to flow into the separator at a different rate and/or for a separated blood component to be removed from the separator at a different rate and/or for the drive unit to be spun at a different speed.

In accordance with the present disclosure, the system and more particularly the controller 300 is programmed or configured to receive inputs from the operator and calculate procedure settings such as flow rates, duration that optimize the collection or therapy. To that end, the controller is programmed or configured to include a "procedure estimator" that receives inputs from the medical practitioner, administrator or system operator and based on such input calculates and/or otherwise determines optimized procedure settings for the collection or therapy at hand.

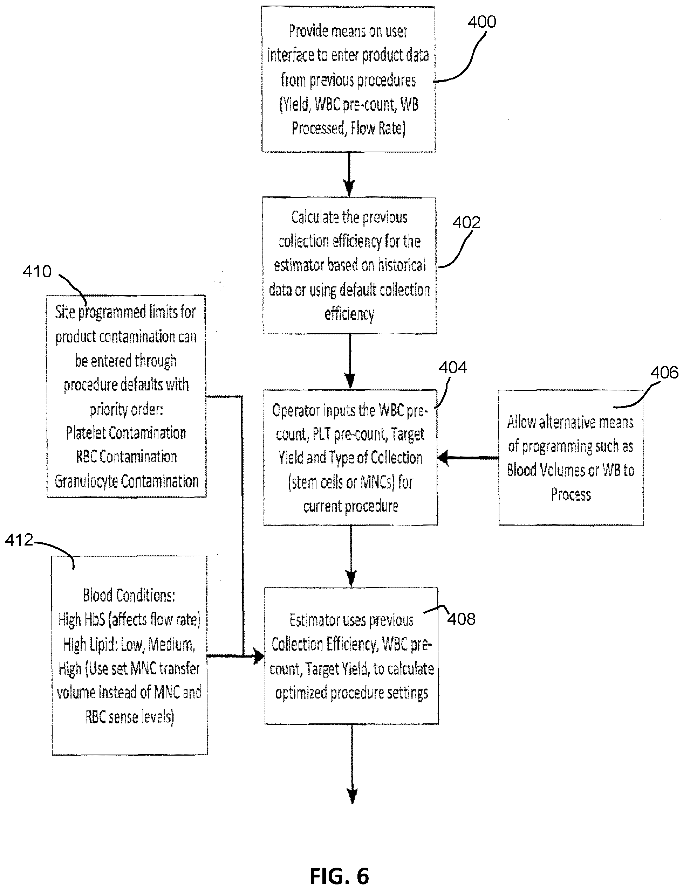

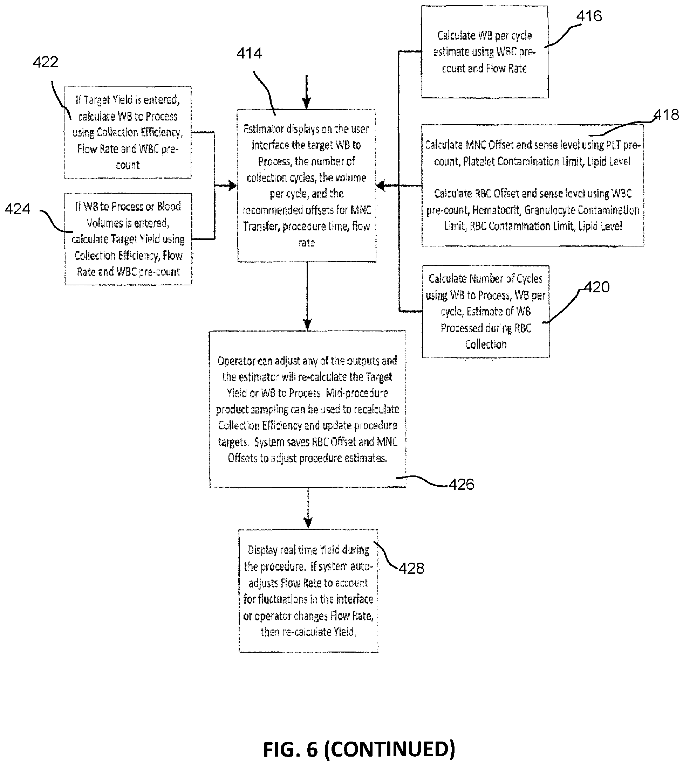

Turning now to the methods for optimizing the collection and/or treatment of blood components using the automated system of the type described above, as noted, separator 10 preferably includes a (graphical) user interface 302 (which may be embodied as video screen 308, shown in Fig 1 and diagrammatically depicted in Fig. 5.) User interface 302 allows the operator or administrator to input data useful in optimizing the given collection or treatment procedure. For example, as shown in block 400 of Fig. 6, the operator may input data from a previous collection or therapy of the donor or patient. Historical data that may be useful in optimizing the procedure and input by the operator/administrator may include, but is not limited to, product yield, white blood cell (WBC) pre-count, volume of whole blood processed and flow rates. Alternatively, rather than inputting the data at the outset of a new procedure, historical data may be stored in the memory of the controller and/or retrieved by the controller from a separately maintained donor/patient database. Regardless, the historical data may be used by the controller to calculate the collection efficiency of previous procedures, as indicated in block 402. If no historical data is available or is simply not used, a default collection efficiency stored in the procedure estimator of the controller may be retrieved and used.

Data regarding the current procedure is entered as shown in block 404 of Fig. 6. This includes, for example, the type of collection procedure to be performed such as a mononuclear cell collection or a stem cell collection. In addition, the operator may input one or more of the white blood cell (WBC) pre-count, platelet pre-count and a Target Yield of the desired component. These entries may be useful in optimizing the collection of a selected component. For example, in the collection of MNCs, a "high" or "low" platelet pre-count may require adjustments in certain "Offset" volumes to collect as many target cells as possible while minimizing contamination of the collected target cells by other components. "Offset" volumes refer to predetermined volumes that the system, under the direction of the controller, uses to regulate the opening and closing of valves on the tubing/flow paths of cassettes 23L, 23M, 23R or elsewhere that leads to the collection container based on detecting (e.g., by optical sensor) the flow/harvesting of blood components from the separation chamber.

In one non-limiting example, if the platelet pre-count is high and the system is programmed to minimize platelet contamination, the MNC Offset setting would be increased to delay the opening of the valve that sends the target cells towards the collection container and allow fewer platelets to enter. Conversely, if the platelet pre-count is low, the MNC Offset setting could be decreased to open the valve earlier in the collection and collect all possible target cells. A high platelet pre-count may also lead to a larger platelet layer. Accordingly, the system may suggest and utilize a larger Red Blood Cell (RBC) Offset to delay the closing of the valve that sends the target cells towards the collection container to allow more target cells to enter. Finally, if the system is operating at a fast separation flow rate with a high platelet count, it may be difficult to keep the interface position stable and retain the target cells in the separation pack. Accordingly, in response to a high platelet count, the system (under the direction of the controller) may reduce the separation flow rate to minimize loss of target cells during the separation phase. Other or alternative parameters that may be input and useful in establishing the recommended procedure settings may include the blood volume or Total Blood Volume of the blood source and/or a volume of whole blood to be processed, as indicated in block 406.

Using the historical or default collection efficiency, white blood cell pre-count, product yield and, optionally, other or alternative data, the procedure estimator of the system will calculate and determine optimized procedure settings (see block 408).

Other conditions may also be considered and input in determining optimized procedure settings. As shown in blocks 410 and 412, contamination of the component being collected by other blood components may influence the procedure settings. For example, in MNC collection procedures, the medical practitioner may be collecting the cells for further processing and the downstream treatment that may require a pre-determined amount of collected cells with acceptable levels of cellular contamination. The collection of MNCs may be subject to contamination by other components that have been separated from whole blood such as red cells, platelets, and/or granulocytes which may affect the overall amount of cells targeted for collection or actually collected. Thus, in accordance with the systems and methods described herein, limits on contamination by other components may be taken into account, balanced against and, where necessary, prioritized over the goal of increasing collection efficiency when calculating/determining optimized procedure settings. For example, as previously discussed, in addressing a high platelet pre-count, the system may be programmed to increase the RBC Offset, which can lead to more RBCs flowing into the collection container. However, strict limits on the red cell content in the collection container exist, then the red cell contamination limit may be prioritized over collection efficiency in determining the value for RBC Offset.

In accordance with further examples, platelet, RBC and granulocyte contamination in an MNC product may have varying effects. If an MNC product is cryopreserved, RBCs are lysed, resulting in free hemoglobin which can cause adverse events during transfusion. ABO type-compatibility is also a concern for allogeneic transplants. Granulocytes can also cause adverse events during transfusion of an MNC product. Platelets can affect the yields/purity of target cells during post-collection selections.

For MNC procedures, the priority is collecting the desired target cell yield, balanced against acceptable product contamination. For example, a large RBC Offset volume can be programmed during harvest to attempt to collect more target cells, however this may result in more RBC and granulocyte contamination in the collected product. For ECP, platelet and granulocyte contamination are less of or not a concern. On the other hand, the RBC contamination (hematocrit) of the collected MNCs to be treated may be of greater concern, as RBCs absorb the UV-A light during photoactivation that is intended for the MNCs. In short, the priority order will be dependent on the MNC product's intended use, specifications and post-collection cell processing procedures.

In general, the amount or level of contamination by other components is an estimate. In accordance with the systems and methods described herein, the amount/volume of RBCs, granulocytes and platelets (contaminants) may be estimated as follows. For example, RBC Offset volume is the determinant factor in RBC contamination in the collection container. The RBC contamination for one MNC harvest cycle can be calculated using the RBC Offset volume. The total RBC contamination volume can be calculated by summing the RBC contamination volumes for all cycles.

For example: y = 0.5567x - 1.7794, where y is RBC Contamination Volume per Cycle and x is RBC Offset Volume. The Total RBC Contamination Volume (mL) = Sum y for all Cycles.

RBC Offset volume may also be used to estimate granulocyte contamination in the collection container, as granulocytes and RBCs are immediately adjacent to each other by density separation. Another factor in estimating the amount of granulocyte contamination is by using the amount of WB processed in each cycle as there is some minor accumulation of granulocytes in the separation pack. Thus, the granulocyte contamination for one MNC harvest cycle can be calculated using the WB Processed per Cycle and RBC Offset volume. The total granulocyte contamination can be calculated by summing the granulocyte contamination for all cycles. For example: where c is the Granulocyte Contamination per Cycle (cells × 10⁹), a is the RBC Offset Volume, and b is the WB processed per Cycle (mL), the Total Granulocyte Contamination (×10⁹) = Sum of c for all cycles.

Factors considered for platelet contamination are MNC Offset, Plasma Storage Fluid Volume of the MNC product, and Patient/Donor Platelet Pre-Count, where p is the Total Platelet Contamination (×10¹¹) for a procedure with two transfer cycles, a is the Plasma Storage Fluid Volume (mL), and b is the Patient/Donor Platelet Pre-count (×10³/µL). Empirical data can be generated using variable MNC Offsets to further optimize the estimation equation.

Other factors may also influence the procedure settings. For example, certain known blood-related conditions may affect procedure collection efficiency, flow rate and product yield. These include diseases or abnormal levels of naturally present compounds that may affect blood separation, such as the presence of lipemia or bilirubin, high WBC counts, high platelet counts, or high hemoglobin HbS (which may affect flow rate). These conditions and their known effects may be factored and input into the calculation of the optimized procedure settings (block 412). For example, high lipemia or bilirubin conditions can slow down the collection of plasma since the system detects the interface between plasma and red cells to be much higher than it is inactuality. It can also affect the optical sensing that initiates the opening and closing of the valve that sends target cells to the collection container. Accordingly, if high lipemic or bilirubin conditions have been input, the system may: (1) use a higher interface setpoint for collection (2) could use pre-set volumes for opening and closing the valve instead of using the optical sensor sense levels, or (3) adjust the optical sensor transmission level for plasma to account for the reduced plasma clarity.

For high WBC counts, the estimator will reduce the volume per cycle to minimize the loss of target cells during the separation phase. For example, if the WBC Count is above 40,000 per microliter, the volume per cycle will be set to 1000 mL. If equal to 40,000 per microliter or below, the volume per cycle will be set to 1400 mL. An alternative response to high WBC count could be a reduction in the separation flow rate to minimize loss of target cells during the separation phase.

High platelet counts may result in the estimator changing the MNC Offset, RBC Offset, or separation flow rate, as previously described.

High hemoglobin S may indicate the presence of abnormally shaped red cells. The presence of abnormally shaped red cells can result in poor separation and lower Hct of the packed red cell bed in the separation pack. This reduction in Hct in the separation pack will not help retain the target cells in the separation pack as the whole blood is processed. To improve the separation efficiency, the estimator may reduce the separation flow rate. The system can also utilize a larger RBC Offset to allow more target cells that may reside deeper in the red cell layer to enter the collection container. Of course, as described above, any adjustments made to Offset values must be balanced against established or accepted contamination levels in the collected component.

Once the optimized procedure settings have been established, the procedure estimator will display some or all of the settings on the graphical user interface 302 (e.g., touch screen)/display 308. Among the settings that may be displayed are the Target Whole Blood To Process, the number of collection cycles, the volume of whole blood to process per cycle, procedure time, flow rate and recommended offsets for mononuclear cells and red blood cells, as noted in block 414 of Fig. 6. It will be noted that the estimated whole blood per cycle may be based on the WBC pre-count obtained from a pre-procedure analysis of the donor's blood and flow on the flow rate (block 416)

With regard to recommended offsets, the procedure estimator calculates a MNC Offset and sense level using, for example, a platelet pre-count, the previously described platelet contamination limit and lipid levels, as shown in block 418. Similarly, an RBC Offset and sense level may be taken into account and calculated using the white blood cell pre-count, hematocrit of the subject, the previously described granulocyte contamination and RBC contamination and lipid levels, as also shown in block 418

The calculated number of collection cycles may be based on the volume of Whole blood to process, the calculated whole blood per cycle (see block 416) and an estimate of whole blood processed during red blood cell collection (block 420),

As shown in blocks 422 and 424, if a target yield is entered, then the Whole blood to process is based on the previously determined Collection Efficiency, flow rate and WBC pre-count (block 422). Where Whole blood to process is entered, the Target yield of cells is calculated using the previously determined Collection Efficiency, flow rate and WBC pre-count (block 424). To calculate the Whole Blood to Process: WB to Process (mL) = Target Yield (cells ×10⁶ /mL) / (Pre Count (cells ×106 /mL) × Collection Efficiency (%) 100%).

Regarding Collection Efficiency, where Pre Count is either Pre CD34+ cells or Pre WBC, Collection Efficiency can be determined by one of the following ways:
1) Average of historical collection efficiency for that patient/donor (either stem cell or MNC) or (2) correlations with collection efficiency (such as WBC pre-count and/or RBC Offset volume). More particularly, for stem cell collection, y = -0.368x + 65.172 where y is collection efficiency, x is Pre WBC when the flow rate is 60 mL/min.

For MNC collection, y = 46.678In(x) - 19.498 where y is collection efficiency, x is RBC Offset.

To calculate the Target Yield, Target Yield (cells ×10⁶ /mL) = WB to Process (mL) × Pre Count (cells ×10⁶ /mL) × Collection Efficiency (%) / 100%

The procedure settings established and recommended by the procedure estimator will be automatically followed under the direction of the controller 300. However, the outputs established by the procedure estimator may be subject to change by the operator. A change in an output setting will result in the procedure estimator recalculating the Target Yield or Whole to process volumes. Also, samples collected from collection containers, such as container 68 (Fig. 3) may warrant a re-calculations of procedure targets/settings or collection efficiencies. Such recalculations use pre-determined MNC and RBC Offsets, as indicated in block 426. Collection of samples in the course of a MNC Collection and/or ECP procedure is described in U.S. Patent Application Publication 2020/0107765 A1.

Graphical user Interface 302/308 will display the product yield in real time during the procedure. Automatic changes to the flow rate (in response to fluctuations in the interface between separated cells in the separation chamber 12 of container 14) may require the system to recalculate the Yield (block 428).

The procedure estimator described herein provides the medical practitioner and/or operator greater flexibility in how they prescribe the collection or treatment by employing recommended settings that better ensure a successful collection/treatment of the targeted cells. The procedure estimator of the present disclosure allows the operator to input product data from previous procedures with the donor/patient, will allow input of known blood conditions that may affect the procedure at hand, establish optimized collection efficiencies while balancing these against and, where necessary, giving priority to certain product contamination limits, and will utilize default collection efficiencies for stem cells or MNCs if no historical data is provided, Finally the procedure estimator will re-calculate outputs if the operator needs to make changes to work within the constraints of the blood source and procedure time limits.

### Other Examples

Aspects of the present subject matter described above may be beneficial alone or in combination with one or more other Aspects, as described below.

Aspect 1. A system for processing and/or treating blood and blood components the system including; a separator for separating blood into its components; a disposable fluid circuit associated with said separator; a controller configured to (i) direct the collection and/or treatment of blood components (ii) store information based on one or more of historical collection data, pre-determined default values or limits for certain components, selected blood component conditions and (iii) establish and/or calculate recommended procedure settings based on said stored information and other input information; and a user interface associated with said separator and coupled to said controller for entering information and displaying said established and/or calculated procedure settings.

Aspect 2. The system of Aspect 1 wherein the processing comprises the collection of stem cells or mononuclear cells (MNCs).

Aspect 3. The system of Aspect 1 wherein the treating comprises extracorporeal photopheresis of mononuclear cells (MNC).

Aspect 4. The system of Aspect 1 wherein the controller is configured to establish and/or calculate recommended procedure settings based at least in part on a determined collection efficiency for a selected component and a selected subject.

Aspect 5. The system of Aspect 4 wherein the collection efficiency is determined based on historical data from previous collections from the subject.

Aspect 6. The system of Aspect 4 wherein the collection efficiency is determined based on a default collection efficiency.

Aspect 7. The system of Aspect 1 wherein the controller is configured to establish and/or calculate recommended procedure settings based at least in part on pre-determined limits on product contamination for a selected blood component.

Aspect 8. The system of Aspect 7 wherein the pre-determined limits on product contamination include limits as to one or more of platelet contamination, red blood cell contamination and granulocyte contamination.

Aspect 9. The system of Aspect 8 wherein the controller is configured to prioritize selected product contamination based on a selected procedure.

Aspect 10. The system of any one of Aspects 1 through 9 wherein the controller is configured to establish and/or calculate recommended procedure settings based at least in part on selected blood conditions.

Aspect 11. The system of Aspect 10 wherein the selected blood conditions include HbS and concentration of lipids.

Aspect 12. The system of any one of Aspects 1 through 11 wherein the controller is configured to receive information of a target blood component yield.

Aspect 13. The system of any one of Aspects 1 through 11 wherein the controller is configured to receive information of a volume of blood to be processed.

Aspect 14. The system of any one of Aspects 1 through 13 wherein the controller is configured to establish and/or calculate one or more of a targeted whole blood volume to process, a blood component targeted yield, a number of collection cycles, a volume per cycle, procedure time, flow rates and recommended offsets for mononuclear cell transfer.

Aspect 15. A method for optimizing a blood component collection and/or treatment procedure in an automated blood cell processing system comprising: entering or retrieving selected product data into said processor; calculating a collection efficiency based on historical data and/or default collection efficiencies; entering selected blood component conditions for a blood component collection and/or treatment procedure; entering or retrieving one or more of product contamination limits, blood conditions; determining recommended procedure parameters based on entered, retrieved, or calculated values from above.

Aspect 16. The method of Aspect 10 further comprising collecting a sample of a selected component and comparing the composition of said sample.

Aspect 17. The method of Aspect 11 further comprising adjusting the procedure settings based on the composition of the collected sample.

Aspect 18. The method of any one of Aspects 15 through 17 comprising selecting the blood component to be collected and/or treated.

Aspect 19. The method of Aspect 18 wherein the blood component to be collected and/or treated is selected from the group of stem cells and mononuclear cells.

Aspect 20. The method of any one of Aspects 15 through 19 wherein said blood conditions include any one or more of HbS, lipid levels, lipemia, bilirubin.

Aspect 21. The method of any one of Aspects 15 through 20 further comprising selecting (i) the component to be collected and (ii) at least one of a white blood cell pre-count, a platelet pre-count, a blood component target yield, a subject blood volume and a volume of whole blood to process.

Aspect 22. The method of Aspect 21 comprising selecting blood product contamination limits for the component to be collected.

Aspect 23. The method of Aspect 22 further comprising determining a prioritized order of blood product contamination for the component to be collected.

Aspect 24. The method of any one of Aspects 22 and 23 wherein the types of contamination include platelet contamination, red blood cell contamination and granulocyte contamination.

Aspect 25. The method of any one of Aspects 15 through 24 further comprising calculating mononuclear cell offset.

Aspect 26. The method of Aspect 25 comprising calculating said mononuclear cell offset using one or more of a platelet count, platelet contamination limit and lipid level.

Aspect 27. The method of any one of Aspects 15 through 26 comprising calculating a red blood cell offset using one or more of a white blood cell pre-count, hematocrit of the subject, granulocyte contamination limit, Red blood cell contamination limit and lipid level.

Aspect 28. The method of any one of Aspects 15 through 27 comprising selecting a target yield of the component to be collected.

Aspect 29. The method of Aspect 28 further comprising calculating the volume of whole blood to process based on a collection efficiency, flow rate and white blood cell pre-count.

Aspect 30. The method of any one of Aspects 15 through 27 comprising a selecting a volume of whole blood to be processed.

Aspect 31. The method of Aspect 30 further comprising calculating the blood component target yield based on a collection efficiency, flow rate and white blood cell pre-count.

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter of the invention.

## Claims

1. A system for processing and/or treating blood and blood components comprising;
a. a separator for separating blood into its components;
b. a disposable fluid circuit associated with said separator;
c. a controller configured to (i) direct collection and/or treatment of blood components (ii) store information based on one or more of historical collection data, pre-determined default values or limits for certain components, selected blood component conditions and (iii) establish and/or calculate recommended procedure settings based on said stored information and other input information; and
d. a user interface associated with said separator and coupled to said controller for entering information and displaying said established and/or calculated procedure settings.

2. The system of Claim 1 wherein said processing comprises the collection of stem cells or mononuclear cells (MNCs).

3. The system of Claim 1 wherein said treating comprises extracorporeal photopheresis of mononuclear cells (MNC).

4. The system of Claim 1 wherein said controller is configured to establish and/or calculate recommended procedure settings based at least in part on a determined collection efficiency for a selected component and a selected subject, wherein said collection efficiency is determined based on historical data from previous collections from the subject or based on default collection efficiency.

5. The system of Claim 1 wherein said controller is configured to establish and/or calculate recommended procedure settings based at least in part on pre-determined limits on product contamination for a selected blood component and/or based at least in part on selected blood conditions.

6. The system of Claim 5 wherein said pre-determined limits on product contamination include limits as to one or more of platelet contamination, red blood cell contamination and granulocyte contamination and wherein said controller is configured to prioritize selected product contamination based on a selected procedure.

7. The system of Claim 6 wherein said selected blood conditions include HbS and concentration of lipids.

8. The system of any one of Claims 1 through 4 wherein said controller is configured to receive information of a target blood component yield and/or a volume of blood to be processed.

9. The system of any one of Claims 1 through 8 wherein the controller is configured to establish and/or calculate one or more of a targeted whole blood volume to process, a blood component targeted yield, a number of collection cycles, a volume per cycle, procedure time, flow rates and recommended offsets for mononuclear cell transfer.

10. A method for optimizing a blood component collection and/or treatment procedure in an automated blood cell processing system comprising:
a. entering or retrieving selected product data into said processing system;
b. calculating a collection efficiency based on historical data and/or default collection efficiencies;
c. entering selected blood component conditions for a blood component collection and/or treatment procedure;
d. entering or retrieving one or more of product contamination limits, blood conditions; and
e. determining recommended procedure parameters based on entered, retrieved, or calculated values from (a)-(d) above.

11. The method of Claim 10 further comprising collecting a sample of a selected component, comparing the composition of said sample, and adjusting the procedure settings based on the composition of the collected sample.

12. The method of any one of Claims 10 through 11 comprising selecting the blood component to be collected and/or treated, wherein said blood component to be collected and/or treated is selected from the group of stem cells and mononuclear cells.

13. The method of any one of Claims 10 through 12 further comprising selecting (i) the component to be collected and (ii) at least one of a white blood cell pre-count, a platelet pre-count, a blood component target yield, a subject blood volume and a volume of whole blood to process.

14. The method of Claim 10 comprising selecting blood product contamination limits for the component to be collected and determining a prioritized order of blood product contamination limits for the component to be collected, wherein the types of contamination include platelet contamination, red blood cell contamination, and granulocyte contamination.

15. The method of any one of Claims 10 through 14 comprising calculating a red blood cell offset using one or more of a white blood cell pre-count, hematocrit of a subject, granulocyte contamination limit, red blood cell contamination limit and lipid level.
